Europäisches Patentamt

European Patent Office

Office européen des brevets

(19)

(11) Numéro de publication: **0 243 390 B1**

(12) **FASCICULE DE BREVET EUROPEEN**

(45) Date de publication du fascicule du brevet: 08.08.90

(51) Int. Cl.⁵: **C 07 K 15/00,** A 61 K 35/16, A 61 K 37/04, G 01 N 33/68

(21) Numéro de dépôt: 86905865.1

(22) Date de dépôt: **14.10.86**

(86) Numéro de dépôt international: PCT/FR86/00353

(87) Numéro de publication internationale: **WO 87/02368 23.04.87 Gazette 87/09**

(54) **NOUVELLE PROTEINE DE MAMMIFERE AYANT UNE ACTIVITE IMMUNOMODULATRICE ET PROGESTATIVE.**

(30) Priorité: 14.10.85 FR 8515196

(43) Date de publication de la demande: **04.11.87 Bulletin 87/45**

(45) Mention de la délivrance du brevet: **08.08.90 Bulletin 90/32**

(84) Etats contractants désignés: **AT BE CH DE FR GB IT LI LU NL SE**

(56) Documents cités: GB-A-1 401 238

C.R. Acad. Sc. Paris, vol. 301, série III, no. 14, 1985, (PARIS; FR); P. Grabar et al.: "Survie prolongée d'allogreffes de peau chez le rat par injection de sérum ou d'une fraction sérique (IRG) etraite de rattes gestantes", pages 653-658

Nature, vol. 256, 7 aou7t 1975, G. Köhler et al.: "Continuous cultures of fused cells secreting antibody of predefined specificity", pages 495-497

(73) Titulaire: **Etablissement Public dit: CENTRE NATIONAL DE LA RECHERCHE SCIENTIFIQUE (CNRS) 15, Quai Anatole France F-75007 Paris (FR)**

(72) Inventeur: **CHATEAUREYNAUD-DUPRAT, Pierrette, Marie Le Videau 350, rue des Girolles St Jean d'Yllac F-33127 Martignas/Jalle (FR)** Inventeur: **GIROUD, Jean-Paul 66, avenue de Breteuil F-75007 Paris (FR)** Inventeur: **GRABAR, Pierre décédé (FR)**

(74) Mandataire: **Tonnellier, Jean-Claude et al Cabinet Nony & Cie. 29, rue Cambacérès F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

## EP 0 243 390 B1

(56) References cited:
Chemical Abstracts, vol. 105, no. 9, 1er septembre 1986, (Columbus, Ohio, US); P. Grabar et al.: "In vitro and in vivo inhibitions of allograft reactions and anticomplement activity by an a2 macroglobulin produced during gestation", & Bul. Acad. Natl. Med. (Paris) 1985, 169(9), 1371-8

J. Repr. Immunol. vol. 6, no. 5, 1984, Elsevier Science Publishers B.V.; M.T. Badet:"Specific and non-specific immunosuppressive factors in salamander pregnancy serum", pages 299-311

Chemical Abstracts, vol. 99, no. 11, 12 septembre 1983, (Columbus, Ohio, US); P. Chauteaureynaud-Duprat et al.:"Inhibition of an immune reaction of hemolysis in higher vertebrates by serium fraction (fraction P) from pregnant Salamandra salamandra L", p. 436, abrégé 86475n, & C.R. Seances Acad. Sci., ser. 3, 1983, 296(7), 323-8

Biological Abstracts, vol. 74, no. 11, 1982, P.C. Duprat et al.: "Inhibition of the in vitro allograft rejection (allograft-induced lymphocytotoxicity) by a fraction extracted from pregnant female serum, in the salamander (Salamandra salamandra)", & C.R. Seances Acad. Sci. Ser. III SI VIE 292(9): 505-598, 1981, abrégé 74398

**Description**

La présente invention à pour objet une nouvelle protéine de mammifère ayant une activité immunomodulatrice et progestative, se préparation et son application notamment comme médicament immunomodulateur, en particulier comme inhibiteur de la réaction de rejet des allogreffes.

On sait que la pratique des greffes d'organes oblige à provoquer artificiellement une inhibition de l'immunité pour éviter la réaction de rejet de l'organe greffé. Cette inhibition des défenses immunologiques est obtenue par l'emploi de diverses méthodes: irradiation, chimiothérapie (agents alkylants, certains antibiotiques tels que la cyclosporine, sérums antilymphocytaires, etc..). Cependant, l'emploi de ces méthodes d'inhibition de l'immunité présente des inconvénients. En effet, ces méthodes paralysent non seulement les défenses de l'organisme contre la greffe, mais aussi les défenses naturelles contre les agents infectieux.

La nouvelle protéine objet de l'invention présente l'avantage de ne pas inhiber les défenses immunitaires autres que l'immunité de greffe.

On sait d'autre part que la gestation représente pour l'immunologiste un paradoxe puisque le foetus, porteur d'antigènes étrangers, et assimilable à une allogreffe, est cependant toléré par la mère pendant toute la gestation.

Ce paradoxe a conduit à étudier le mécanisme immunologique faisant obstacle au rejet du foetus par la mère, et plusieurs chercheurs ont démontré les propriétés immunosuppressives de certaines protéines présentes principalement chez la femelle gestante et normalement absente chez la femelle non gestante et chez le mâle.

C'est ainsi qu'on a décrit une globuline associée à l'état de gestation, ayant un poids moléculaire de l'ordre de 510.000, qui possède des propriétés immunosuppressive in vitro; voir par exemple Stimson et coll. FEBS Letters, Vol. 23, n°3, 298—302 (1972); Stimson, Clin. Ex. Immunol., 25, 199—206 (1976).

On a également décrit une protéine dite PZP (Pregnancy Zone Protein) de poids moléculaire 359.000, liée à l'état hormonal de la gestation, présente également chez les femmes utilisant les pilules contraceptives, et qui inhibe in vitro la stimulation des lymphocytes par la phytohémagglutinine; voir par exemple Von Schoultz et coll., FEBS Letters, Vol. 38, n°1, 23—26 (1973).

On a aussi décrit la dépression de l'activité lymphocytaire in vitro par deux facteurs sériques apparaissant au tout début de la gestation, le premier (poids moléculaire 180.000) apparaissant 6 heures après la fertilisation, la deuxième (poids moléculaire 40.000) apparaissant 4 à 5 jours après chez la souris; voir par exemple Clark et coll., Clin. Exp. Immunol., 32, 318—323 (1978).

La démonstration d'un phénomène de rejet maternel contre l'embryon et de l'inhibition de cette réaction n'a toutefois été faite, jusqu'à présent, que chez un organisme ovovivipare, la salamandre; voir par exemple Chateaureynaud et coll. J. Repr. Immunol., I, 47—60 (1979) et Badet et coll., Ann. Immunol. (Inst. Pasteur) 131D, 57—69 (1980).

On a maintenant découvert une nouvelle protéine, présente chez les mammifères femelles gestantes, qui possède des propriétés, (non spécifiques d'individus voire d'espèces) d'inhibition du rejet des allogreffes, permettant notamment une survie prolongée des allogreffes cutanées, sans modification apparente des autres défenses de l'organisme. On désignera ici cette protéine par le sigle I.R.G. (inhibitrice du rejet de greffes).

La présente invention a pour objet une protéine dite I. R. G. caractérisée par le fait:

qu'elle est présente dans le sérum des femelles gestantes et normalement absente chez les mâles;

qu'elle est présente dans le sérum des individus développant une réaction inflammatoire;

qu'elle migre en électrophorès avec les alpha$_2$-macroglobulines;

qu'elle a una masse moléculaire, mesurée par électrophorèse sur gel de polyacrylamide, de 800.000±50.000 environ;

qu'elle réagit spécifiquement avec des anticorps dirigés contre la fraction alpha$_2$-macroglobuline des femelles gestantes;

qu'elle inhibe de façon significative in vitro l'activité des lymphocytes cytotoxiques au cours d'une réaction lymphocytaire mixte secondaire;

que, mélangé avec le complément, elle inhibe la réaction d'hémolyse des globules rouges de moutons sensibilisés;

qu'apres incubation avec le complément, elle n'a pas d'activité antiprotéase;

et que son administration in vivo prolonge de façon significative la survie des allogreffes; ainsi que les analogues de cette protéine.

Dans la présente demande, on désigne par "analogues" de la protéine I. R. G. tous fragments et tous peptides synthétiques ou semi-synthétiques contenant des séquences peptidiques présentes sur la protéine I. R. G. ainsi que tous dérivés de ces fragments ou peptides, à conditions que ces fragments, peptides ou dérivés satisfassent aux conditions suivantes:

ils inhibent de façon significative l'activité in vitro des lymphocytes cytotoxiques au cours d'une réaction lymphocytaire mixte secondaire; et

leur administration in vivo prolonge de façon significative la survie des allogreffes.

Il convient de remarquer que, contrairement à l'I.R.G., le sérum entier de femelle gestante n'a pas d'activé anti-complément et n'a pas d'activité anti-protéases, à l'inverse des fractions sériques des individus autres que les femelles gestantes ou autres que les individus présentant des réactions inflammatoires. En outre l'I.R.G. n'inhibe pas les mécanismes de défense humorale (formation d'anticorps).

L'invention a également pour objet un procédé de purification et d'obtention de la protéine I.R.G., ce procédé étant caractérisé par le fait:

que l'on fractionne le sérum de mammifères femelles gestantes, et/ou d'animaux mammifères développant une réaction inflammatoire expérimentale, de façon à isoler des fractions migrant avec les alpha$_2$-macroglobulines;

et que l'on soumet lesdites fractions à un épuisement immunologique avec des anticorps IgG anti-sérum entier de mâle et/ou anti-(alpha$_2$-macroglobulines) et mâle pour recueillir les fractions ne réagissant pas avec lesdits anticorps,

étant entendu que l'on effectue les diverses opérations de fractionnement et de purification dans une solution tampon ayant un pH de 7,6 à 8,1.

On a en effet constaté que c'est dans cette gamme de pH que l'on peut isoler la protéine IRG ayant les propriétés biologiques annoncées ci-dessus, et que ces propriétés biologiques peuvent être conservées.

Les tampons utilisables peuvent être facilement déterminés par des expériences de routine. Un tampon préféré est constitué par un mélange de phosphates monosodique et disodique (par exemple 0,0M) contenant NaCl 0,20—0,30M. De préférence, on prépare le tampon phosphate en mélangeant les phosphates monosodique et disodique, puis on ajuste le pH à 8 environ. On ajoute ensuite le chlorure de sodium jusqu'à la molarité désirée, puis on ajuste le pH à la valeur finale désirée (de 7,6 à 8,1 et de préférence de 7,7 à 8,0).

Le sérum de femelle gestante ne contient la protéine I.R.G. qu'après un certain temps, variable et facilement déterminable pour chaque espèce, après la fertilisation. Chez la ratte, ce temps est de l'ordre de 12 jours. Chez la femme, l'activité d'I.R.G. est croissante jusqu'au 3$^e$ mois de la grossesse puis reste stable jusqu'à 10 jours après l'accouchement.

Les méthodes de fractionnement utilisées peuvent comprendre toute méthode connue de fractionnement des protéines du sérum comme par exemple la filtration sur gel, l'ultracentrifugation, le relargage par les sels neutres, la précipitation fractionnée à l'aide d'un agent précipitant (tel qu'un alcool, un polyalkylène- glycol, ou le Rivanol), la chromatographie d'adsorption, la chromatographie sur résine échangeuse d'ions, la chromatographie d'affinité, etc...

Le principe de l'utilisation de ces méthodes de fractionnement des protéines est connu et ne sera pas rappelé ici.

La filtration sur gel est effectuée par exemple sur gel de dextrane ou de polyacrylamide.

Parmi les sels neutres utilisés pour le relargage, on citera par exemple le sulfate d'ammonium et les phosphates alcalins.

La chromatographie d'adsorption peut être effectuée sur des supports tels que l'alumine, le phosphate de calcium, la silice, etc.,

Les résines échangeuses d'ions sont par exemple des celluloses modifiées telles que la diéthylaminoéthyl cellulose ou la carboxyméthyl cellulose.

On peut également utiliser la chromatographie d'affinité avec un support pulvérulent inerte sur lequel on fixe par covalence, selon les méthodes connues, soit des anticorps dirigés contre la protéine I.R.G., soit des anticorps dirigés contre une fraction alpha$_2$-macroglobulines de femelle gestante, soit des anticorps dirigés contre la fraction alpha$_2$-macroglobulines de mâle. Dans les deux premiers cas, la protéine I.R.G. se fixe sur le support, dans l'autre cas elle reste dans l'éluat.

Pour concentrer la protéine obtenue on peut utiliser, outre les techniques de précipitation, la technique d'ultrafiltration.

La technique d'épuisement immunologique consiste à mettre en contact la fraction protéique à purifier avec des anticorps dirigés contre au moins une fraction protéique analogue (par exemple une fraction d'alpha$_2$-macroglobulines) provenant du sérum d'un mâle, puis à recueillir la fraction protéique qui ne se fixe pas sur lesdits anticorps. Bien entendu, on peut utiliser des anticorps dirigés contre le sérum total de mâle.

L'invention a également pour objet l'utilisation de la protéine I.R.G. ou de ses analogues, ou de fractions sériques la contenant, comme ingrédient actif dans un médicament immunomodulateur ayant notamment la propriété d'inhiber l'immunité de greffe sans affecter les autres défenses de l'organisme, ou dans un médicament progestatif.

Le médicament de l'invention est utilisable en particulier dans la prévention et le traitement des malades recevant des greffes d'organes.

Le médiacement de l'invention peut être administré notamment par voie parentérale ou locale.

A cet effet, il peut être présenté par exemple sous la forme de solutions injectables ou de poudres lyophilisées pour solutions injectables, ou encore sous la forme d'implants ou de réservoirs.

Dans le cas d'une transplantation d'organe, l'administration du médicament de l'invention peut être effectuée selon des modalités diverses en fonction des techniques de dépression immunitaire utilisées par le médecin avant et après la greffe.

Le médicament de l'invention peut être administré avant la transplantation d'organes pour provoquer la dépression de l'immunité de greffe, et/ou après la transplantation, en relais ou avec d'autre médications

EP 0 243 390 B1

suppressives de l'immunité.

La posologie dépend notamment du mode d'adminis ration et de la phase du traitement. Par exemple, chez les humains, elle varie généralement de 0,1 à 10 g de principe actif par jour chez l'adulte.

On a découvert que chez les mammifères femelles faisant des avortements spontés, la protéine I.R.G. est souvent absente. Il est possible de favoriser le maintien de la gestation chez ces individus par administration d'I.R.G.

La protéine IRG peut également être utilisée pour favoriser l'implantation de l'oeuf, notamment dans le cas d'une fécondation *in vitro*.

L'invention a également pour objet d'utilisation de la protéine I.R.G. ou de ses analogues dans la préparation d'un médicament immunomodulateur ou progestatif.

Les études effectuées ont montre que l'I.R.G. apparaît dans le sérum non seulement lors de la gestation mais aussi, plus généralement, chez les mâles comme chez les femelles, lors de toute réaction inflammatoire.

On conçoit donc l'intérêt des anticorps dirigés soit contre des fractions sériques contenant la protéine I.R.G., soit contre la protéine I.R.G. purifiée, soit encore contre des déterminants antigéniques caractéristiques de cette protéine. En effet, non seulement ces anticorps peuvent servir à la purification ou à la détection de la présence ou de l'absence de la protéine I.R.G., mais aussi l'administration de ces anticorps permettra d'inhiber, lorsqu'elle est indésirable, la tolérance, acquise ou potentielle, vis-à-vis des alloantigènes, induite par l'I.R.G. apparaissant dans le sérum lors des processus inflammatoires. Les anticorps anti-I.R.G. ou les anticorps analogues apparaissent donc comme des agents thérapeutiques nouveaux permettant la prévention et le traitement des maladies consécutives à des processus inflammatoires et conduisant à des tolérances anormales vis-à-vis des alloantigènes, telles que les maladies de système, les maladies auto-immunes, et certains types de cancers, etc...

L'invention a donc pour objet des anticorps dirigés contre des fractions sériques contenant la protéine I.R.G., contre la protéine I.R.G., contre des analogues de cette protéine (tels que définis plus haut), ou contre des déterminants antigéniques caractéristiques de cette protéine (y compris des anticorps monoclonaux).

Ces anticorps seront appelés ici "anticorps anti-I.R.G.", et englobent bien entendu les fragments Fab ou $F(ab')_2$ correspondants obtenus par clivage enzymatique.

L'invention s'étend aux anticorps anti-I.R.G. tels que définis précédemment mais modifiés par marquage avec un traceur radioactif, fluorescent ou enzymatique, obtenu selon les méthodes classiques de préparation de tels anticorps marqués.

Par exemple, le traceur peut être un traceur radioactif obtenu par échange isotopique avec un isotope radioactif, par exemple de l'iode ou de l'indium. Le couplage des anticorps avec un agent traceur radioactif est connu en soi et peut être réalisé par exemple selon la méthode de GREENWOOD.

Le couplage des anticorps avec un fluorochrome (par exemple isothiocyanate de fluorescéine ou de rhodamine) est bien connu.

Le couplage des anticorps avec un traceur enzymatique est également connu en soi et peut être réalisé par exemple en fixant l'enzyme sur les molécules d'immunoglobulines selon une méthode analogue à celle décrite par NAKANE, Journal Histochemistry Cytochemistry, 22:1984—1991 (1974).

L'enzyme est par exemple une peroxydase ou une phosphatase alcaline.

L'activité enzymatique éventuellement présente sur le réactif, après réalisation d'un test, peut être déterminée selon les méthodes connues avec un substrat approprié permettant par exemple une révélation par colorimétrie, par fluorescence, par luminescence, par potentiométrie, etc....

L'invention s'étend également aux anticorps anti-I.R.G. monoclonaux obtenus selon la technique connue des hybridomes au départ des lymphocytes prélevés sur des animaux immunisés selon la méthode qui sera décrite ci-après.

L'invention s'étend en outre aux anticorps anti-I.R.G. polyclonaux on monoclonaux, marqués ou non, fixés sur un support solide permettant leur utilisation comme agents immunoadsorbants dans des méthodes de chromatographie d'affinité ou comme réactifs dans les méthodes d'analyse fondées sur la réaction antigène-anticorps (techniques radioimmunologiques, techniques d'immunofluorescence, techniques immunoenzymatiques).

Ledit support solide peut être réalisé avec tout matériau solide, biologique ou synthétique, doué de propriétés adsorbantes ou capable de fixer un agent de couplage. Ces matériaux sont connus et décrits dans la littérature. Parmi les matériaux solides capables de fixer les anticorps par adsorption, on citera par exemple le polystyrène, le polypropylène, les latex, etc... Parmi les matériaux solides utilisables pour fixer les anticorps par covalence à l'aide d'un agent de couplage, on peut citer notamment le dextran, la cellulose, leurs dérivés aminés (diéthylamino-cellulose ou diéthylamino-dextran), etc...

Le support solide peut se présenter par exemple sous forme de disques, de tubes, de baguettes, de billes, ou de plaques de microtitration.

Les agents de couplage permettant de fixer par covalence les anticorps sur le support solide sont des dérivés bifonctionnels tels que des dialdéhydes, des quinones, etc...

Les anticorps peuvent également être fixés sur des supports solides minéraux, par exemple selon les méthodes décrites dans les brevets français 2.319.176, 2.403.556 et 3.403.098.

L'invention a également pour objet un procédé de préparation des anticorps tels que définis ci-dessus.

5

Ce procédé est principalement caractérisé par le fait que l'on immunise des animaux par administrations répétées, selon les méthodes connues, d'I.R.G., de fractions sériques enrichies en I.R.G., ou de fragments antigéniques d'I.R.G éventuellement couplés à une macromolécule, puis que l'on recueille les anticorps sériques desdits animaux immunisés, et que, si désiré, on fixe lesdits anticorps sur un support selon les méthodes connues et/ou les fait réagir avec un marqueur radioactif, fluorescent ou enzymatique selon les méthodes connues et/ou vérifie la spécificité des anticorps obtenus. Pour recueillir les anticorps sériques, on utilise les techniques classiques de fractionnement permettant d'isoler les immunoglobulines.

Pour obtenir l'I.R.G. animale destinée à l'immunisation, on peut induire chez l'animal la formation d'I.R.G. en provoquant une réaction inflammatoire expérimentale.

L'invention a également pour objet l'utilisation des anticorps anti-I.R.G. comme agents de purification ou de détection de l'I.R.G., comme agents anti-projestatifs, et aussi comme médicaments dans la prévention et le traitement des maladies consécutives à des processus inflammatoires et caractérisées par une tolérance anormale vis-à-vis des allo-antigènes. Ces anticorps peuvent être administrés par voie parentérale ou locale, les doses étant les doses usuelles pour les anticorps.

Les anticorps anti-I.R.G., éventuellement fixés sur un support, sont utilisables comme réactifs dans toutes les techniques fondées sur la réaction antigène-anticorps, notamment les dosages radioimmunologiques, les techniques d'immunofluorescence, les techniques immuno-enzymatiques, les techniques d'immunodiffusion et les techniques d'immunoélectrophorèse.

Par exemple, pour détecter ou doser l'I.R.G. et/ou un de ses analogues en solution, on peut utiliser les techniques immunoenzymatiques connues (notamment un test ELISA). On met en contact la solution à tester avec un support adsorbant de façon à adsorber l'antigène (I.R.G.), s'il est présent. On ajoute une première solution d'anticorps spécifiques anti-I.R.G. tels que ceux définis ci-dessus. On rince et on ajoute ensuite une deuxième solution d'anticorps, qui sont cette fois des anti-immunoglobulines marquées par un traceur enzymatique, ces anti-immunoglobulines étant dirigées contre les anticorps de l'espèce animale ayant servi à préparer les anticorps de ladite première solution. On rince à nouveau le support.

Il suffit ensuit de révéler la présence des anticorps marquées éventuellement fixés sur le support pour savoir si l'I.R.G. était présente ou non.

Les médicaments à base d'anticorps anti-I.R.G. sont administrés par voie parentérale, locale, etc. A cet effet, ils sont présentés par exemple sous la forme de solutions injectables, de préparations lyophilisées pour solutions injectables, de pommades, l'implants, etc.

Il convient de noter aussi qu'il est possible de déterminer la présence, la diminution ou l'absence de l'I.R.G. chez les mammifères, et en particulier chez la femme enceinte, en mesurant l'activité biologique de l'I.R.G. sur des fractions sériques purifiées, par exemple par chromatographie et/ou par épuisement immunologique. L'activité biologiqué étudiée peut être en particulier l'inhibition du complément (test de la lyse immune) ou encore l'inhibition de la cytotoxicité (par exemple après une réaction lymphocytaire mixte secondaire).

On peut ainsi réaliser un test de prédiction des risques d'avortement chez des sujets gestants, en particulier chez la femme entre les troisième et quinzième semaines de la grossesse.

Pour cela, on compare l'activité d'I.R.G. d'une fraction sérique purifiée dudit sujet à celle des sujets gestants normaux. Il y a risque d'avortement lorsque l'activité d'I.R.G. est fortement diminuée, par exemple d'au moins 60%.

On peut également réaliser le test de cytotoxicité en remplaçant la réaction lymphocytaire mixte secondaire par deux greffes successives de peau chez le rat. On étudie ensuite la cytotoxicite des cellules spléniques du receveur (qui peuvent être cultivées notamment sur du sérum d'homme AB) sur les cellules du donneur (lymphocytes traités à la mitomycine ou mieux macrophages péritonéaux, marqués au chrome). On peut ainsi réaliser un essai de cytotoxicité en 18 heures, au lieu de 7 jours avec la technique classique décrite par Châteaureynaud et al., Annales Gynécologiques, pp. 181—189 (1982).

Il faut remarquer enfin que l'I.R.G. d'inflammation apparaît à des moments différents selon le type d'inflammation, et que le moment de l'activité inhibitrice maximale de la cytotoxicité ou du complément ne correspond pas au moment où la quantité des alpha$_2$-macroglobulines est maximale (voir la partie expérimentale). L'I.R.G. pourrait donc correspondre à une modification locale d'une protéine d'inflammation existante.

Les exemples suivants illustrent l'invention sans toutefois la limiter.

EXEMPLE 1. Purification de la protèine I.R.G. de rat

I. ANIMAUX

Les animaux utilisés sont des rats de souche pure WISTAR/FURTH, FISCHER/344 (IFFA-CREDO), WAG (fournis par l'Institut de recherche sur le cancer de VILLEJUIF), pesant 150—200 grammes.

II. SERIES EXPERIMENTALES

A. GESTATION ALLOGENIQUE: (Femelle FISCHER/344 X mâle WISTAR/FURTH on WAG).

On appelle jour 1 de la gravidité, celui de la mise en évidence d'un bouchon vaginal ou de la présence des spermatozoïdes dans le frottis vaginal le lendemain de la mise au mâle. Le sant est prélevé aux 6e, 9e, 15e et 19e jours de la gestation et 1er, 3e, 5e et 7e jours du post-partum.

6

B. DECIDUOME EXPERIMENTAL: (Femelles pseudogestantes)

L'état de la pseudogestation est induit chez des rattes FISCHER/344 par ligature utéro-tubaire au 2e jour de la gestation pour empêcher les ovules fécondés de descendre dans les cornes utérines et de s'implanter. L'équilibre hormonal progestérone-oestrogène est celui de la gestation vraie. La réaction déciduale est obtenue par traumatisme mécanique chez ces femelles pseudogestantes par insertion le 5e jour (jour de l'implantation de l'oeuf chez la femelle gestante d'un fil dans chaque corne utérine. Le sang est prélevé les 6e, 9e, 12e jours de la pseudogestation.

C. INDUCTION D'UNE REACTION INFLAMMATOIRE "AIGUE"

L'enrichissement sérique en alpha$_2$-macroglobuline a été réalisé chez le mâle FISCHER/344 normal par injection sous-cutanée bilatérale de 2×0,5 cm$^3$ d'essence de térébenthine (induction d'une inflammation "aiguë") associée à l'administration intra-musculaire de 5 mg d'acétate de cortisone. Le sérum inflammatoire est prélevé 48h après ces injections.

III. FILTRATION SUR GEL

La filtration sur gel de polyacrylamide commercialisé sous la marque Ultrogel® AcA$_{22}$ (IBF) de gamme de fractionnement (100 000—1 200 000) a été utilisée. La colonne 100×1,6 cm (LKB) a été avec un tampon phosphate 0.05M, NaCl 0,25M, pH 8,0.

Le calibrage de la colonne a été effectué avec des protéines de référence.

Un volume de 2 cm$^3$ de sérum de rat est déposé sur la surface du gel par au adaptateur lié à une pompe péristaltique (volume total du gel: 180 cm$^3$).

Les différents sérums subissent auparavant une dialyse contre le tampon de départ (tampon phosphate 0.05M, NaCl, 0,25M, pH 8,0) pendant 2 heures.

L'élution est effectuée dans le même tampon phosphate pH 8,0. Le volume de chaque fraction recueillie est de 5 ml.

On mesure l'absorption à 280 nm des fractions éluées, à l'aide d'un spectrophotomètre.

Les fractions sériques (I, II, III, IV, etc...) recueillies sont concentrées jusqu'au volume initial (2 ml.), par ultra-filtration sur membrane Millipore® 100×M (Amicon).

La fraction IV correspond au deuxième pic observé au spectrophotomètre lors de l'élution.

IV. ANALYSE ELECTROPHORETIQUE EN GEL DE POLYACRYLAMIDE. Résultats

Du 12e jour de gestation au 7e jour post-partum, le sérum de femelles allogestantes et le sérum de femelles en post-partum montrent la présence d'une bande protéique supplémentaire dans la fraction IV (zone de gammaglobulines IgM).

Le sérum d'inflammation "aiguë" de mâle et le sérum de femelle pseudogestante ont montré une bande protéique supplémentaire dans la fraction IV, qui est comparable à celle observée dans le sérum de femelle allogestante.

La fraction IV contient l'ensemble des alpha$_2$-macroglobulines.

V. DETERMINATION DES POIDS MOLECULAIRES

Le poids moléculaire de la bande protéique caractéristique de la gestation est déterminé par la comparaison de la mobilité électrophorétique en gel avec celle des protéines de référence.

Cette bande protéique a une mobilité $R_F$ 0,11 et son poids moléculaire est approximativement 840.000 Daltons.

VI. EPUISEMENT IMMUNOLOGIQUE

Immunisation de lapins contre la fraction IV

La fraction sérique IV (50—200 µg) de mâle normal FISCHER/344 est mélangée avec l'adjuvant de Freund complet.

L'émulsion qui en résulte est injectée par voie intra-dermique. Les injections sont répétées sous les dix jeurs par voie intra-musculaire à dose protéique de 50 µg en adjvant de Freund incomplet.

Le prélèvement de sang a lieu une semaine après la quatrième injection.

Les IgG spécifiques de l'immun sérum obtenu sont précipités par le sulfate d'ammonium et purifiés sur DEAE cellulose.

On prépare de façon analogue un immun sérum de lapin contenant des IgG dirigées contre la fraction sérique IV de rattes non gestantes.

TECHNIQUE D'EPUISEMENT IMMUNOLOGIQUE

100 µl d'antigène (fraction sérique) de femelle allogestante sont ajoutés à 300 µl d'immun sérum spécifique (IgG). Le mélange est laissé 1 heure à température ambiante et 2 heures à +4°C., puis centrifugé 15 minutes à 3000 g.

Le procédé est répété en ajoutant 1 volume d'immunsérum à 1 volume de surnageant. Après une nuit à +4°C, on centrifuge 15 minutes à 3000 g, et on recueille le surnageant.

On opère de même avec les fractions sériques de femelle pseudogestante et de mâle développant une inflammation aiguë.

## VII. CARACTERISATION DE LA PROTEINE

### a) IMMUNO-DIFFUSION EN GELOSE

Après épuisement immunologique soit de la fraction IV de femelle allogestante, soit de la fraction IV du sérum de femelle pseudogestante (déciduome) soit de la même fraction de sérm d'inflammation "aiguë" par l'immun-sérum anti-(fraction IV) de mâle normal, on l'observe plus qu'un seul arc de précipitation pour la fraction sérique IV épuisée de femelle allogestante ou pour la fraction sérique IV épuisée de femelle porteuse d'un déciduome ou de mâle ayant subi une inflammation "aiguë".

### b) ANALYSE IMMUNO-ELECTROPHORETIQUE

Les résultats précédents sont confirmés par l'analyse immuno-électrophorétique.

Les fractions sériques IV (de femelle gestante ou pseudo-gestante, ou d'inflammation aiguë) épuisées avec l'immun-sérum anti-fraction IV de mâle montrent un seul arc de précipitation principale.

### c) ETUDE ELECTROPHORETIQUE

La fraction sérique IV épuisée de femelle allogestante avec l'immun sérum anti-fraction IV de mâle est étudiée par électrophorèse en gel de polyacrylamide (gradient de 4 à 12%), qui révèle une seule bande protéique d'une mobilité $R_F$ 0,17 et de poids moléculaire 760 000 Daltons, comparable aux fraction sériques IV épuisées de décidualisation et d'inflammation.

## EXEMPLE 2:

En opérant de façon analogue à celle décrite à l'exemple 1, au départ d'un pool de sérums de femmes enceintes (entre le 3e et le 9e mois de grossesse), on a obtenu une fraction protéique analogue à la fraction IV de l'exemple 1.

Par épuisement immunologique avec des anticorps de lapin dirigés contre le sérum total d'homme et avec des anticorps de lapin dirigés contre la fraction sérique d'alpha$_2$-macroglobuline de femmes non gestantes, on a obtenu une protéine I.R.G. purifiée ayant des caractéristiques analogues à celles de la protéine décrite à l'exemple 1.

En outre, chez 11 femmes en menace d'avortement sans cause connue (de 3 semaines à 3 mois de grossesse) on a observé l'absence d'activité d'I.R.G. dans les sérums individuels.

Chez la femme, on a observé la présence de la protéine I.R.G. dans la période de 2 à 8 heures qui suit l'ovulation sans fécondation, ainsi que dans la période de 5 à 8 heures précédant la menstruation.

## EXEMPLE 3: Etude de la protéine I.R.G.:
## INHIBITION "IN VITRO" DE LA REPONSE T CYTOTOXIQUE ALLOSPECIFIQUE

La réponse T cytolytique se développe au cours d'une réaction lymphocytaire mixte (RLM) secondaire unidirectionelle. Pour cela, des cellules spléniques sont prélevées chez des rats mâles Fischer, 5 jours après la pose d'un greffon de peau de rat Wag. Ces cellules immunisées ($2 \times 10^6$) sont incubées en présence d'un même nombre de cellules spléniques de rat Wag traitées à la mitomycine C (cellules stimulantes), dans un volume total de 2 ml de RPMI 1640 (Eurobio) additionné de tampon HEPES (20 mM), de 2-mercaptoéthanol ($5 \times 10^{-5}$ M) d'antibiotiques, et de 5% de sérum de rat inactivé par la chaleur. Après 7 jours de culture à 37°C dans une atmosphère humide contenant 5% de $CO_2$, l'activité cytolytique des cellules répondeuses est étudiée en CML (lympholyse à médiation cellulaire) contre des cellules péritonéales de rat Wag préalablement stimulées par la phytohémagglutinine et marquées au Chrome 51, le rapport cellules tueuses/cellules cibles étant de 100/l. Après une incubation de 6 heures à 37°C, la quantité de chrome 51 relarguée dans le surnageant est mesurée et le pourcentage de cytotoxicité calculé d'après la formule:

$$\frac{\text{cpm expérimental—cpm spontané}}{\text{cpm incorporé sur les cellules cibles}} \times 100$$

La fraction sérique examinée pour son activité immunosuppressive est ajoutée à une concentration finale de 5% (100 µl) soit au début de la culture lymphocytaire mixte (temps zéro), soit après le 7e jour de la culture durant la dernière heure de l'incubation. Dans les deux cas, les cellules sont lavées avant la mesure de leur activité cytolytique. Le pourcentage d'inhibition de la cytotoxicité est calculé d'après la formule:

$$100 - \frac{\text{\% cytotoxicité en présence de la fraction sérique}}{\text{\% cytotoxicité en absence de la fraction sérique}}$$

Les fractions sériques examinées sont la fraction IV du sérum de rat mâle développant une inflammation aiguë, la même fraction purifiée (par épuisement immunologique), et la fraction IV purifiée de femelle gestante. Les résultats sont résumés dans les tableaux I et II ci-après.

Des résultats analogues ont été obtenus avec les fractions sériques de rattes femelles pseudogestantes et de femmes enceintes.

TABLEAU I*

| Fractions seriques testees | No. Exp. | | | | % De Cytotoxicite | % D' Inhibition |
|---|---|---|---|---|---|---|
| | 1 | 2 | 3 | 4 | | |
| Témoins | 46.28 | 56.48 | 56.46 | 50.46 | 52.42±4.97 | |
| Fr. IV  T | 16.87 | 14.07 | 17.25 | 12.72 | 15.22±2.40 | 70.96±1.70 |
| Fr. IV  TP | 12.92 | 10.90 | 15.15 | 15.85 | 13.70±1.12 | 73.86±1.25 |
| Fr. IV  gP | 11.22 | 12.32 | 14.50 | 13.22 | 12.81±0.75 | 75.56±0.75 |

\* Les fractions sériques purifées sont ajoutées au temps 0 de la RLM.
Résultats significatifs $p < 0.001$
Fr. IV T: fraction IV d'inflammation aiguë
Fr. IV gP: fraction IV purifiée de femelle gestante
Fr. IV TP: fraction IV purifiée d'inflammation aiguë

TABLEAU II*

| Fractions seriques testees | No. Exp. | | | | % De Cytotoxicite | % D' Inhibition |
|---|---|---|---|---|---|---|
| | 1 | 2 | 3 | 4 | | |
| Témoin | 46.28 | 56.48 | 56.46 | 50.46 | 52.42±4.97 | — |
| Fr. IV  T | 18.88 | 18.30 | 18.95 | 16.10 | 18.05±0.66 | 65.56±0.50 |
| Fr. IV  TP | 18.15 | 15.10 | 17.10 | 13.22 | 15.89±1.09 | 69.68±1.22 |
| Fr. IV  gP | 13.37 | 13.90 | 15.45 | 17.98 | 15.17±0.90 | 70.06±0.75 |

\* Les fractions fractions sériques purifiées sont ajoutées au jour 7 de la RLM
résultats significatifs $p < 0.001$
Fr. IV T: fraction sérique d'inflammation aiguë
Fr. IV gP: fraction IV purifiée de femelle gestante
Fr. IV TP: fraction IV purifiée d'inflammation aiguë

EXEMPLE 4: Etude de l'inhibition de l'activité du complément *in vitro*

On utilisé le sérum de rattes allogestantes Fischer/344 accouplées à des mâles Wistar, prélevé aux jours 9, 12, 15 et 19 de la gestation, ainsi que des sérums de femmes enceintes depuis 3 à 9 mois et de femmes enceintes en préavortement spontané.

Pour extraire et purifier les fractions correspondant à la protéine IRG, on opère comme à l'exemple 1.

L'inhibition du complément a été mesurée par la méthode classique de l'hémolyse en plaques de microtitration.

RESULTATS:

Le sérum entier ou des fractions des sérums de rats femelles allogestantes ou de femmes enceintes ajoutés au milieu contenant des hématies de mouton sensibilisées par des IgG de lapin anti-mouton n'a pas d'activité inhibitrice sur le complément de cobaye. L'incubation préalable (1h à 37°C) de ces sérums entiers ou du sérum entier de mâle ne modifie pas la réaction de lyse immune. Mais après incubation préalable de la fraction I.R.G. avec le complément, on constate que:

1) il y a inhibition de l'activité complémentaire par la fraction IV et par la fraction I.R.G.;

2) il y a augmentation de l'activité anti-complémentaire de ces fractions après purification;

3) la fraction correspondant à l'I.R.G. des sérums de rats mâles, de rats femelles vierges ou de femmes enceintes sur le point d'avorter n'entraîne pas l'inhibition de l'activité complémentaire (à dilutions comparables).

On a recherché en outre si la protéine I.R.G. possède une activité anti-protéase. Lorsque l'I.R.G. est incubée avec le la trypsine et un petit substrat (le T.A.M.E.) ou un gros substrat (caséine), on constate que:

9

1) l'I.R.G. n'inhibe pas l'activité de la trypsine mais au contraire l'augmente;

2) l'I.R.G. après incubation avec la trypsine et ses substrats, garde ses propriétés anti-complémentaires;

3) la fraction de sérum de rat mâle correspondant à l'I.R.G. inhibe l'activité de la trypsine; elle a donc une activité anti-protéase.

L'I.R.G. de gestation serait donc un activateur des protéases induisant comme la plasmine soit l'activation de $C_1S$ dans la voie classique et du $C_3$ dans les voies classiques et alterne. Cette propriété n'est trouvée que dans le sérum de femelles gestantes normales.

En outre, on a observé:

que le sérum entier d'homme, de femme enceinte multipare (2ème à 8ème mois) et de femme en avortement spontané n'inhibe pas la réaction d'hémolyse immune;

que la fraction IV d'homme (AB) n'inhibe pas cette réaction, contrairement à la fraction IV de femme enceinte (en particulier de 3 semaines à 15 semaines).

EXEMPLE 5: Etude *in vivo*:
INHIBITION DU REJET D'ALLOGREFFES CUTANEES, AVEC MAINTIEN DES DEFENSES IMMUNITAIRES AUTRES QU'ANTI-GREFFES.

Des rats Fisher/344 reçoivent le jour 0 un greffon de peau de rat Wag de 1 cm de diamètre dans la région sous scapulaire. Le greffon étant maintenu par six points de suture, le jour du rejet est défini comme celui où le greffon nécrosé se détache de son lit (14,5 jours après la pose du greffon chez les témoins).

Les receveurs de l'allogreffe sont traités avec au total 0,5 ml de la fraction sérique répartis en plusieurs injections intraveineuses effectuées avant et après la greffe.

Les résultats sont résumés dans le Tableau III ci-après.

En administrant la même quantité d'I.R.G. répartie en plusieurs injections (8 injections intra-veineuses aux jours J−3, J−2, J−1, J+3 et J+5 à J+7, +3 injections locales sous le greffon à J+5 et J+7), le temps moyen de survie du greffon a été de 30,60±0,30 jours (limites extrêmes des temps de rejet: 29 à 31 jours).

En administrant la même quantité d'I.R.G. répartie en plusieurs injections (7 injections intraveineuses aux jours J−3, J−2, J−1, J+1, J+3, J+5, J+6 et 4 injections locales de J+7 à J+10), les limites extrêmes des temps de rejet ont été de 45 à 51 jours.

Des expériences préliminaires sur la qualité des défenses de l'organisme avant et après une allogreffe chez le rat avec ou sans injection d'I.R.G.:

injection de glycoprotéines de membrane de *T. equiperdum* (Botat 1), réputée comme stimulant les défenses humorales,

infestation avec un parasite non mortel (*Plasmodium berghei* berghei),

ont montré que les défenses ne sont pas modifiées lorsque l'allogreffe est précédée et suivie d'injections d'I.R.G.

On rappellera ici que chez le rat l'infestation par *Plasmodium berghei* berghei est une maladie curable et que la résistance à la maladie fait appel aux défenses immunitaires cellulaire et humorale.

TABLEAU III

| Traitement | No. allogreffe | Limites extremes des temps de rejet de greffe | Temps moyen de survie± ecart type |
|---|---|---|---|
| Sérum physiologique (témoins) | 10 | 13—15 | 14.50±0.34 |
| Fr. IV g | 10 | 17—21 | 20.71±1.01 |
| Fr. IV T | 10 | 21—27 | 24.00±0.58 |

Fr. IV g: fraction sérique purifiée de femelle gestante
Fr. IV T: fraction sérique purifiée d'inflammation aiguë
Résultats significatifs: $p < 0,001$

EXEMPLE 6: Préparation d'anticorps anti-I.R.G.

On immunise des lapins contre la protéine I.R.G. de ratte obtenue à l'exemple 1 (fraction IV purifiée par épuisement immunologique).

La technique d'immunisation et de purification des gammaglobulines est analogue à celle décrite à l'exemple 1.

On obtient une préparation de gammaglobulines anti-(I.R.G. de ratte) permettant par exemple de purifier ou de détecter cette protéine I.R.G.

En outre, on a administre à des rattes gestantes les anticorps ainsi préparés (2 fois 1 ml de solution d'anticorps à 6g/litre au jours J3 et J5 de la gestation). Cette administration a provoqué des avortements entre J6 et J10 dans les cinq cas étudiés.

EXEMPLE 7:

Cinétique de l'enrichissement sérique en alpha$_2$-macroglobulines par induction d'une réaction inflammatoire aiguë.

a) INDUCTION D'UNE REACTION INFLAMMATOIRE AIGUE.

On effectue cette étude sur des rats Sprague Dawley mâles agés de 6 semaines.

On provoque la réaction inflammatoire expérimentale soit par injection sous-cutanée bilatérale de $2 \times 0,5$ cm$^3$ d'essence de térébenthine, soit par l'injection d'une suspension de 1 ml à 1% (poids/volume) de cristaux de pyrophosphate de calcium dans la plèvre.

Les sérums sont prélevés à divers intervalles de temps après le déclenchement de la réaction inflammatoire. Chaque résultat est donné pour un pool de sérums de cinq animaux injectés.

b) FRACTIONEMENT DES SERUMS PAR FILTRATION SUR GEL

La technique utilisée est celle décrite ci-dessus à l'exemple 1.III.

La solution tampon utilisée ici est un tampon phosphate 0,05M, NaCl 0,25M, pH 7,8.

Avant d'être déposés sur la colonne, les sérums sont dialysés contre ledit tampon pendant au moins 4 heures à 5°C.

Les éluats sortant de la colonne passent devant une cellule optique reliée à un enregistreur de densité optique à 280 nm, puis sont recueillis par fractions de 3,5 ml. Les fraction correspondant à chaque pic sont réunies.

Après élution, les fractions sont concentrées par ultrafiltration dans une cellule Amicon pourvue d'une membrane XM 100, jusqu'au volume de départ.

La fraction analogue à la fraction IV de l'exemple 1 (deuxième pic) est recuillie.

La concentration des protéines est déterminée par la méthode de Bradford, avec l'ovo-albumine comme protéine de référence.

Les résultats sont résumés dans le Tableau IV ci-après. L'enrichissement en alpha$_2$-macroglobulines n'est pas concomitant avec l'activité inhibitrice maximale, différente selon les modèles utilisés (essence de térébenthine, pyrophosphate de calcium) comme le montrent les exemples suivants:

TABLEAU IV

| Sérum | Concentration en protéines (µg/µl de la fraction IV) |
|---|---|
| Témoins | 3,6 |
| Traitement à la térébenthine | |
| Sérum prélevé au boute de: | |
| 6h | 3,15 |
| 24h | 3,5 |
| 40h | 4,15 |
| 48h | 5,15 |
| 72h | 2,7 |
| 5 jours | 4,35 |
| Traitement au pyrophosphate | |
| Sérum prélevé au bout de: | |
| 2h | 2,0 |
| 6h | 1,26 |
| 24h | 2,28 |
| 48h | 2,42 |
| 72h | 4,20 |
| 5 jours | 3,5 |

EXEMPLE 8:

PURIFICATION DE LA FRACTION ALPHA$_2$-MACROGLOBULINES DE FEMME ENCEINTE

Les sérums sont obtenus par prélèvement de sang de femmes enceintes.

Les fractions sériques d'alpha$_2$-macroglobulines de chacun des sérums sont séparées par filtration sur gel, de façon analogue à celle décrite dans l'exemple 1. On observe, par rapport au sérum de rat, l'apparition d'un pic supplémentaire. On recueille les fractions correspondant à la fraction IV de l'exemple 1, c'est-à-dire la fraction qui, dans les mêmes conditions, sort au bout du même temps. Il s'agit ici du troisième pic, alors que chez le rat la fraction IV correspondait au deuxième pic. Par analogie, ce troisième pic est encore appelé ici "fraction IV".

EXEMPLE 9

ANALYSE PAR ELECTROPHORESE DES SERUMS ET DES FRACTIONS SERIQUES

Les sérums prélevés chez des rats en réponse inflammatoire (exemple 7) et chez des rats témoins, chez des femmes à différents stades de la grossesse (exemple 8) et chez des hommes (témoins), ainsi que les fractions sériques d'alpha$_2$-macroglobulines correspondantes (exemples 7 et 8) ont été analysés par électrophorèse en gradient de gel de polyacrylamide.

Diverses solutions tampon ont été employées concurremment:

tampon Tris-glycine qui permet une bonne révélation de l'ensemble des protéines sériques;

tampon Tris-acide borique-EDTA qui permet une meilleure résolution des constituants alpha$_2$-macroglobulines.

Les électrophorèses ont été réalisées à voltage assez élevé (200 volts) et pendant une longue durée (18 heures) à basse température (4°C) de manière à stabiliser chaque protéine selon sa masse moléculaire propre.

On a utilisé ici trois types de gradients d'acrylamide: 4—12% pour l'ensemble des protéines sériques, 4—15% pour les sérums humains et 4—8% pour assurer une meilleure définition dans le cas des hauts poids moléculaires, et notamment de la fraction IV.

Les résultats mis en évidence par l'analyse électrophorétique sont les suivants:

Rats soumis à une réaction inflammatoire expérimentale:

Une bande protéique de poids moléculaire supérieur à celui de l'alpha$_1$-macroglobuline apparaît en quantité progressivement croissante au cours de l'évolution de la réponse inflammatoire.

Les résultats obtenus avec les sérums d'inflammation au pyrophosphate de calcium et à l'essence de térébenthine sont similaires, seule la cinétique du phénomène est différente.

Sérum de femme enceinte, en comparaison avec un sérum d'homme:

Dans la zone correspondant à la fraction IV, il existe une bande protéique chez l'homme (sérum AB$^+$) mais cette bande est plus importante chez la femme enceinte (grossesse normale).

Le poids moléculaire de cette protéine est de 760.000 environ.

EXEMPLE 10

TEST DE CYTOTOXICITE

Ce test est effectué, de façon analogue à celle décrite à l'exemple 3, avec le sérum entier et les fractions sériques correspondantes riches en IRG des rats mâles ayant subi la réaction inflammatoire expérimentale (exemple 7).

Dans le cas de la réaction inflammatoire provoquée par l'essence de térébenthine, le sérum entier ne donne pas lieu à une inhibition de cytotoxicité spécifique.

Au contraire, les fractions sériques riches en IRG provenant des sérums prélevés ù 24 heures, 48 heures et 72 heures après l'inflammation provoquée donnent lieu à une inhibition de la cytotoxicité spécifique. Dans ce cas, la mesure de cytotoxicité est réalisée en utilisant le marquage au chrome 51 des cellules cibles. L'activité est maximum 48 heures après l'inflammation.

Les autres fractions sériques (obtenues par filtration sur gel; voir exemples 1 et 7) n'inhibent pas la cytotoxicité dans ce test.

Le sérum de femme enceinte (du 3ème mois de la grossesse jusqu'à 10 jours après l'accouchement), inhibe la réaction cytotoxique cellulaire *in vitro*. Le sérum de femmes en risque d'avortement n'a pas ces propriétés.

EXEMPLE 11

TESTS D'INHIBITION DU COMPLEMENT PAR LE SERUM INFLAMMATOIRE DE RAT

On a utilisé la réaction d'hémolyse immune pour tester la capacité d'inhibition du complément de cobayes par des sérums entiers et des fractions sériques de rats.

La réaction d'hémolyse immune consiste à mettre en présence des hématies de mouton sensibilisées par des IgG de lapin anti-hématies de mouton, avec une source de complément.

Sensibilisation des hématies

Une suspension d'hématies standardisées en tampon Mayer (tampon Véronal, Mg$^{2+}$ Ca$^{2+}$ pH 7,2) est sensibilisée par un égal volume d'une solution titrée de sérum hémolytique. Le mélange est laissé pendant au moins 20 minutes à température ambiante, puis placé à 4°C jusqu'à utilisation extemporanée.

Source de complément

Il s'agit d'un mélange de sérums de 20 cobayes fractionnés en aliquotes de 50 µl.

Test en tubes

On étudie l'inhibition de la réaction de l'hémolyse immune par des quantités décroissantes de la fraction étudiée.

Pour cette étude on prépare une série de tubes contenant 100 µl de complément (sérum de cobaye) à une dilution fixe qui est ici de 1/400 (CH 50=2.130), 100 µl de la fraction étudiée, à différentes concentrations. On laisse incuber pendant 1 heure à 37°, puis on ajoute 200 µl de la suspension d'hématies de moutons sensibilisés, à $10^8$ cellules/ml. On laisse incuber pendant 45 minutes à 37°C, puis on arrête la réaction. Après centrifugation pendant 5 minutes à 800g, on effectue une lecture au spectrophotomètre à 413 nm.

Tests en plaques de microtitration

On emploie des microplaques Microwell NUNC de 96 cupules à fond rond.

On effectue des dilutions en série du sérum ou des fractions à tester dans des tampons Mayer pH 7,2.

On verse dans chaque puits 20 µl de la fraction étudiée et 40 µl de complément dilué ici au 1/300. On laisse incuber pendant 1 heure à 37°C puis on ajoute 40 µl de la suspension d'hématies sensibilisées. On laisse incuber pendant 45 minutes à 37°C avec agitation, puis on centrifuge pendant 5 minutes à 800 g. On lit les résultats de l'hémolyse par estimation à l'oeil nu.

Les fractions ou les sérums entiers testés sont recueillis en tampons phosphate et sont testés directement.

Dans l'espèce humaine (contrairement au rat) la dialyse de la fraction contre le tampon Mayer pH 7,2 augmente la sensibilité du test.

On a également testé la capacité d'inhibition d'inhibition du complément de cobaye par les fractions alpha$_2$-macroglobulines en faisant varier la concentration du complément par rapport à une quantité fixe de protéines.

Résultats

Tous les sérums entiers obtenus au cours de la réaction inflammatoire provoquée par les cristaux de pyrophosphate ou par injection d'essence de térébenthine chez les rats mâles Spraque Dawley, ainsi que les sérums entiers des rats mâles témoins ne sont pas inhibiteurs de la réaction d'hémolyse par le complément *in vitro*.

Au contraire, toutes les fractions sériques d'inflammation riches en I.R.G. (alpha$_2$-macroglobulines; fraction IV ayant subi le traitement d'épuisement immunologique) inhibent l'action du complément *in vitro*.

On a constaté qu'il existe une cinétique du développement du pouvoir inhibiteur du complémentaire pour les fractions provenant des sérums de rats soumis à une réaction inflammatoire expérimentale.

Dans la reaction inflammatoire provoquée par l'essence de térébenthine, le pouvoir inhibiteur apparaît après 6 heures de réaction, atteint une valeur maximum au bout de 24 heures environ et commence ensuite à décroître.

Dans la réaction inflammatoire provoquée par le pyrophosphate, l'inhibition se manifeste à partir de 48 heures de réaction et atteint une valeur maximum vers 72 heures environ.

L'inhibition provoquée par l'essence de térébenthine est plus forte.

Les autres fractions sériques obtenues par filtration sur gel (exemples 1 et 7) ne donnent pas lieu à cette réaction d'inhibition.

Pour quantifier les résultats, on a défini une unité arbitraire I 50 qui est la quantité de protéines capables de produire 50% d'inhibition de 1 µl de la source de complément dans une réaction d'hémolyse immune. Bien entendu, les unités I 50 ne peuvent être comparées que dans le cas d'une même source de complément, sinon, il faut les rapporter au CH 50 de la source de complément utilisé.

On a défini ensuite une "activité spécifique" qui est le nombre d'unités I 50/mg de la fraction sérique étudiée.

Les résultats sont résumés dans le Tableau V suivant:

# EP 0 243 390 B1

## TABLEAU V

| | Echantillon | I 50 | Activite specifique |
|---|---|---|---|
| - | Echantillon | I 50 | Activite specifique |
| | Témoins | 25.0 | 40 |
| Inflammation à la térébenthine Sérum prélevé au bout de: | | | |
| | 6h | 7,4 | 135 |
| | 24h | 2,5 | 400 |
| | 40h | 12,5 | 80 |
| | 72h | 6,3 | 158 |
| | Echantillon | I 50 | Activite specifique |
| | Témoins | 29 | 34,4 |
| Inflammation au pyrophosphate | | | |
| | 24h | 18 | 55,5 |
| | 48h | 11 | 90,9 |
| | 72h | 9 | 111,0 |
| | 120h | 13 | 76,9 |

## Revendications

1. Protéine I.R.G. (inhibitrice du rejet des greffes) purifiée caractérisée par le fait:

qu'elle est présente dans le sérum des mammifères femelles gestantes et normalement absente chez les mâles;

qu'elle est présente dans le sérum des individus développant une réaction inflammatoire;

qu'elle migre en électrophorèse avec les $alpha_2$-macroglobulines;

qu'elle a une masse moléculaire, mesurée par électrophorèse sur gel de polyacrylamide, de $800.000\pm50.000$ environ;

qu'elle réagit spécifiquement avec des anticorps dirigés contre la fraction $alpha_2$-macroglobuline des femelles gestantes;

qu'elle inhibe de façon significative *in vitro* l'activité des lymphocytes cytotoxiques au cours d'une réaction lymphocytaire mixte secondaire;

que, mélangée avec le complément, elle inhibe la réaction d'hémolyse des globules rouges de moutons sensibilisés;

qu'après incubation avec le complément, elle n'a pas d'activité antiprotéase;

et que son administration *in vivo* prolonge de façon significative la survie des allogreffes;

ainsi que les analogues de cette protéine, lesdits analogues étant tous fragments et tous peptides synthétiques ou semi-synthétiques contenant des séquences peptidiques présentes sur la protéine I.R.G. ainsi que tous dérivés de ces fragments ou peptides, à conditions que ces fragments, peptides ou dérivés satisfassent aux conditions suivantes:

ils inhibent de façon significative l'activité *in vitro* des lymphocytes cytotoxiques au cours d'une réaction lymphocytaire mixte secondaire;

leur administration *in vivo* prolonge de façon significative la survie des allogreffes.

2. Procédé de purification et d'obtention de la protéine I.R.G., caractérisé par le fait:

que l'on fractionne le sérum de mammifères femelles gestantes, et/ou d'animaux mammifères développant une réaction inflammatoire expérimentale, de façon à isoler des fractions migrant avec les $alpha_2$-macroglobulines;

et que l'on soumet lesdites fractions à un épuisement immunologique avec des anticorps IgG antisérum entier de mâle et/ou anti-($alpha_2$-macroglobulines) de mâle pour recueillir les fractions ne réagissant pas avec lesdits anticorps;

étant entendu que l'on effectue les diverses opérations de fractionnement et de purification dans une solution tampon ayant un pH de 7,6 à 8,1.

3. Utilisation de la protéine I.R.G. ou de ses analogues, ou de fractions sériques la contenant, comme ingrédient actif dans un médicament immunomodulateur ayant notamment la propriété d'inhiber l'immunité de greffe sans modifier de façon notable les autres défenses de l'organisme, ou dans un médicament progestatif.

14

4. Utilisation de la protéine I.R.G. ou de ses analogues comme ingrédient actif dans la préparation d'un médicament immunomodulateur ou progestatif.

5. Anticorps anti-I.R.G. caractérisé par le fait qu'ils sont dirigés contre la protéine I.R.G. ou contre ses analogues tels que définis dans la revendication 1, ou contre des déterminants antigéniques caractéristiques de cette protéine, lesdits anticorps, polyclonaux ou monoclonaux, étant éventuellement marqués et/ou fixés sur un support solide permettant leur utilisation comme agents immunoadsorbants dans des méthodes d'analyse fondées sur la réaction antigène-anticorps.

6. Procédé de préparation des anticorps de la revendication 5, caractérisé par le fait que l'on immunise des animaux par administrations répétées d'I.R.G., selon les méthodes connues, de fractions sériques enrichies en I.R.G., ou de fragments antigéniques d'I.R.G. éventuellement couplés à une macromolécule, puis que l'on recueille les anticorps sériques desdits animaux immunisés, et que, si désiré, on fixe lesdits anticorps sur un support selon les méthodes connues et/ou les fait réagir avec un marqueur radioactif, fluorescent ou enzymatique selon les méthodes connues et/ou vérifie la spécificité des anticorps obtenus.

7. Utilisation des anticorps anti-I.R.G. tels que définis dans la revendication 5, comme agents de purification ou de détection de l'I.R.G., comme agents anti-progestatifs, ou comme ingrédients actifs dans des médicaments pour la prévention et le traitement des maladies consécutives à des processus inflammatoires.

8. Médicament caractérisé par le fait qu'il contient comme ingrédient actif la protéine I.R.G. ou ses analogues, tels que définis dans la revendication 1, ou des anticorps anti-I.R.G.

9. Procédé de réalisation d'un test de dépistage des risques d'avortement chez un sujet gestant, caractérisé par le fait que l'on mesure l'activité biologique d'I.R.G sur une fraction sérique dudit sujet, purifiée par chromatographie et/ou par épuisement immunologique, et que l'on compare cette activité à celle présente chez les sujets gestants normaux.

## Patentansprüche

1. Gereinigtes, die Transplantatabstoßung hemmendes Protein (IRG-Protein), dadurch gekennzeichnet, daß es

im Serum von weiblichen schwangeren Säugetieren vorhanden ist und normalerweise bei den männlichen Tieren nicht vorkommt,

im Serum von Individuen mit einer entzündlichen Reaktion vorhanden ist,

in der Elektrophorese mit den $\alpha_2$-Macroglobulinen wandert,

eine Molekülmasse, bestimmt durch Elektrophorese auf Polyacrylamidgel, von etwa 800.000±50.000 hat,

spezifisch mit Antikörpern, die gegen die $\alpha_2$-Macroglobulinfraktion der schwangeren weiblichen Tiere gerichtet sind, reagiert,

in vitro die Aktivität von zytotoxischen Lymphozyten im Verlauf einer gemischten sekundären lymphozytären Reaktion deutlich hemmt,

beim Vermischen mit dem Komplement die Hämolysereaktion der roten Blutkörperchen von sensibilisierten Schafen hemmt,

nach der Inkubation mit dem Komplement keine Antiprotease-Aktivität aufweist, und

nach seiner in vivo-Verabreichung die Überlebenszeit der Allotransplantate deutlich verlängert,

sowie Analoge dieses Proteins, wobei diese Analoge alle synthetische oder halbsynthetische Fragmente und Peptide sind, die auf dem IRG-Protein vorhandene Peptid-Sequenzen enthalten, sowie alle Derivate dieser Fragmente oder Peptide, unter der Voraussetzung, daß diese Fragmente, Peptide oder Derivate folgende Bedingungen erfüllen:

sie hemmen deutlich die in vitro-Aktivität der zytotoxischen Lymphozyten im Verlauf einer gemischen sekundären lymphozytären Reaktion,

ihre in vivo-Verabreichung verlängert deutlich die Überlebenszeit der Allotransplantate.

2. Verfahren zur Reinigung und Isolierung des IRG-Proteins, dadurch gekennzeichnet, daß

das Serum von weiblichen schwangeren Säugetieren und/oder Säugetieren mit einer experimentellen entzündlichen Reaktion so fraktioniert wird, daß die mit den $\alpha_2$-Macroglobulinen wandernden Fraktion isoliert werden, und

diese Fraktionen einer immunologische Erschöpfungsreaktion mit IgG-Antikörpern eines männlichen Anti-Vollserums und/oder männlichen Anti($\alpha_2$-Macroglobulinen) unterworfen werden, um Fraktionen, die mit diesen Antikörpern nicht reagieren, zu sammeln, wobei die verschiedenen Fraktionierungs- und Reinigungsstufen in einer Pufferlösung mit einem pH-Wert von 7,6 bis 8,1 durchgeführt werden.

3. Verwendung des IRG-Proteins oder seiner Analoga, oder es enthaltender Serumfraktionen als Wirkstoff in einem immunomodulatorischen Medikament, das insbesondere die Fähigkeit aufweist, die Immunität von Transplantaten zu hemmen, ohne die anderen Abwehrkräfte des Organismus deutlich zu verändern, oder in einem trächtigkeitsfördernden Medikament.

4. Verwendung des IRG-Proteins oder seiner Analoga als Wirkstoff bei der Herstellung eines immunomodulatorischen oder trächtigkeitsfördernden Medikaments.

5. Anti-IRG-Antikörper, dadurch gekennzeichnet, daß sie gegen das IRG-Protein oder seine Analoga nach Anspruch 1 oder gegen charakteristische Antigen-Determinanten dieses Proteins gerichtet sind, wobei die polyklonalen oder monoklonalen Antikörper ggf. markiert und/oder auf einem festen Träger fixiert sind, wodurch ihre Verwendung als immunoabsorbierende Mittel in auf der Antigen-Antikörper-Reaktion beruhenden Analysenverfahren möglich ist.

6. Verfahren zur Herstellung der Antikörper nach Anspruch 5, dadurch gekennzeichnet, daß Tiere in bekannten Verfahren durch wiederholte Verabreichung von IRG-Protein, mit IRG-Protein angereicherten Serumfraktionen oder IRG-Protein-Antigen-Fragmenten, die ggf. an ein Macromolekül gekoppelt sind, immunisiert werden, die Serum-Antikörper der immunisierten Tiere gesammelt werden und, wenn es erwünscht ist, die Antikörper auf einem Träger nach bekannten Verfahren fixiert und/oder nach bekannten Verfahren mit einem radioaktiven, fluoreszierenden oder enzymatischen Marker umgesetzt werden und/ oder die Spezifität der erhaltenen Antikörper geprüft wird.

7. Verwendung der Anti-IRG-Antikörper nach Anspruch 5 als Reinigungs- oder Nachweismittel für das IRG-Protein, als trächtigkeitshemmende Mittel oder als Wirkstoff in Medikamenten zur Verhinderung oder Behandlung von Erkrankungen, die auf entzündliche Prozesse folgen.

8. Medikament, gekennzeichnet durch den Gehalt eines IRG-Proteins oder seiner Analoga nach Anspruch 1 oder an Anti-IRG-Antikörper als Wirkstoff.

9 Verfahren zur Durchführung eines Nachweistests der Risiken einer Fehlgeburt bei einer schwangeren Person, dadurch gekennzeichnet, daß die biologische Aktivität des IRG-Proteins in einer durch Chromotographie und/oder immunologische Erschöpfungsreaktion gereinigten Serumfraktion der Person bestimmt und mit der Aktivität bei normalen schwangeren Personen verglichen wird.

**Claims**

1. A purified "G.R.I." (graft-rejection-inhibitory) protein, characterized by the fact:

that it is present in the serum of pregnant female mammals and normally absent among males;

that it is present in the serum of individuals developing an inflammatory reaction;

that in electrophoresis it migrates with the alpha$_2$-macroglobulins;

that is has a molecular mass, measured by electrophoresis on polyacrylamide gel, of about 800.000±50.000;

that it reacts specifically with antibodies directed against the alpha$_2$-macroglobulin fraction from pregnant females;

that in vitro it significantly inhibits the activity of the cytotoxic lymphocytes in the course of a mixed secondary lymphocytic reaction;

that mixed with the complement it inhibits the reaction of haemolysis of the red corpuscles of sensitized sheep;

that after incubation with the complement it has not antiprotease activity;

and that its administration in vivo significantly prolongs the survival of allografts;

as well as the analogues of this protein, the said analogues being any fragments and any synthetic or semisynthetic peptides containing peptidic sequences present on the G.R.I. protein as well as any derivatives from these fragments or peptides on condition that these fragments, peptides or derivatives satisfy the following conditions:

in vitro they significantly inhibit the activity of the cytotoxic lymphocytes in the course of a mixed secondary lymphocytic reaction;

their administration in vivo significantly prolongs the survival of allografts.

2. A method of purification and of obtaining the G.R.I. protein, characterized by the fact:

that the serum from pregnant female mammals and/or from mammalian animals developing an experimental inflammatory reaction is fractionated so as to isolate fractions which migrate with the alpha$_2$-macroglobulins;

and that the said fractions are subjected to an immunological breakdown with male whole antiserum IgG antibodies and/or male anti(alpha$_2$-macroglobulins) in order to collect the fractions which do not react with the said antibodies;

it being understood that the diverse operations of fractionation and purification are carried out in a buffer solution having a pH of from 7.6 to 8.1.

3. Utilization of the G.R.I. protein or its analogues or serum fractions containing it, as the active ingredient in an immunomodulatory medicine having in particular the property of inhibiting the graft immunity without significantly modifying the other defenses of the system, or in a progestational medicine.

4. Utilisation of the G.R.I. protein or its analogues as the active ingredient in the preparation of an immunomodulatory or progestational medicine.

5. Anti-G.R.I. antibodies characterized by the fact that they are directed against the G.R.I. protein or against its analogues as defined in Claim 1, or against antigenic determinants characteristic of this protein the said polyclonal or monoclonal antibodies being if necessary marked and/or fixed upon a solid support enabling their utilization as immunoadsorbent agents in methods of analysis based upon the antigen-antibody reaction.

6. A method of preparation of the antibodies as in Claim 5, characterized by the fact that animals are immunized by repeated administrations of G.R.I. in accordance with known methods, of serum fractions enriched with G.R.I. or antigenic fragments of G.R.I. possibly coupled to a macromolecule, that then the serum antibodies are collected from the said immunized animals, and that if desired the said antibodies are fixed on a support in accordance with known methods or are made to react with a radioactive, fluorescent or enzymatic marker in accordance with known methods and/or the specificity of the antibodies obtained is checked.

7. Utilization of the anti-G.R.I. antibodies as defined in Claim 5, as agents for purification or detection of G.R.I., as antiprogestational agents or as active ingredients in medicines for the prevention and treatment of diseases consequent upon inflammatory processes.

8. A medicine characterized by the fact that it contains as its active ingredient the G.R.I. protein or its analogues as defined in Claim 1, or anti-G.R.I. antibodies.

9. A method of effecting a test for detecting the risks of abortion in a pregnant subject, characterized by the fact that one measures the biological activity of G.R.I. upon a serum fraction from the said subject, purified by chromatography and/or by immunological breakdown, and that this activity is compared with that present in normal pregnant subjects.